# DEMANDE DE BREVET EUROPEEN

(11) **EP 2 022 532 A1**
(43) Date de publication de la demande: **11.02.2009**
(21) Numéro de dépôt: 07113826.7
(22) Date de dépôt: 06.08.2007
(51) Int. Cl.: A61N 1/378, A61N 1/08, A42B 1/24, A42B 1/06

(54) **Dispositif de recharge de la source d'énergie d'un implant crânien**

(71) Demandeur: CSEM Centre Suisse d'Electronique et de Microtechnique SA Recherche et Développement, 2002 Neuchâtel (CH)
(72) Inventeur: Perrin, Bernard, 2013, Colombier (CH); Decotignie, Jean-Dominique, 1033, Cheseaux (CH)
(74) Mandataire: GLN

(57) **Abrégé**

L'invention concerne un dispositif de recharge de la source d'énergie électrique d'un implant crânien par transfert inductif transcutané d'énergie entre au moins une bobine primaire (12) et une bobine secondaire associée à l'implant. Ce dispositif comporte, en plus de la bobine primaire, des moyens pour la positionner et la maintenir en regard de la bobine secondaire et une station de base (14) pour commander son alimentation. Selon l'invention, la bobine primaire (12) est disposée dans une calotte (10) destinée à emboîter le crâne du patient.

## Description

La présente invention se rapporte au domaine de la médecine. Elle concerne, plus particulièrement, un dispositif de recharge de source d'énergie électrique d'un implant crânien.

Un tel type d'implant est destiné au traitement de diverses pathologies, telles que la maladie de Parkinson, l'épilepsie et le cancer. Il est essentiellement constitué d'une électronique de commande, d'une source d'énergie rechargeable et d'un actuateur pouvant prendre la forme d'une simple électrode de stimulation du cerveau ou d'une micro-pompe délivrant des doses de médicament.

Un problème important qui se pose est la recharge à distance de la source d'énergie de l'implant. Le document US 2005/0004619 propose d'intégrer dans un casque audio classique un module de transfert d'énergie par induction. L'implant comporte alors une bobine secondaire répondant à la bobine primaire du casque, lequel est doté de moyens permettant d'ajuster la position de sa bobine pour optimiser sa relation avec celle de l'implant.

Le résultat est un casque audio alourdi et rendu peu discret par les moyens d'ajustement de sa position qui, en plus, augmentent son coût.

La présente invention a pour but de fournir un dispositif de recharge exempt de ces inconvénients.

De façon plus précise, l'invention concerne un dispositif de recharge de la source d'énergie électrique d'un implant crânien, par transfert inductif transcutané d'énergie entre au moins une bobine primaire et une bobine secondaire associée à l'implant. Ce dispositif comporte, en plus de la bobine primaire, des moyens pour la positionner et la maintenir en regard de la bobine secondaire et une station de base pour commander son alimentation. Selon l'invention, la bobine primaire est disposée dans une calotte destinée à emboîter le crâne du patient.

De manière avantageuse, le dispositif selon l'invention comporte une pluralité de bobines primaires réparties sur une surface suffisamment grande pour que l'une de ces bobines se trouve de manière optimale en regard de la bobine de l'implant. Dans ce cas, la station de base comporte des moyens permettant, grâce à une communication sans fil, de sélectionner et activer la bobine primaire la mieux couplée avec la bobine de l'implant.

D'autres caractéristiques de l'invention ressortiront de la description qui va suivre, faite en regard du dessin annexé dans lequel :
- la figure 1 représente schématiquement la tête d'un patient coiffée d'une casquette de recharge de la source d'énergie de son implant crânien ;
- la figure 2 montre, vue de dessus, une casquette possédant plusieurs bobines primaires décalées les unes par rapport aux autres ; et
- la figure 3 donne un schéma de principe des moyens utilisés, dans le mode de réalisation de la figure 2, pour identifier la bobine primaire la mieux centrée sur la bobine de l'implant.

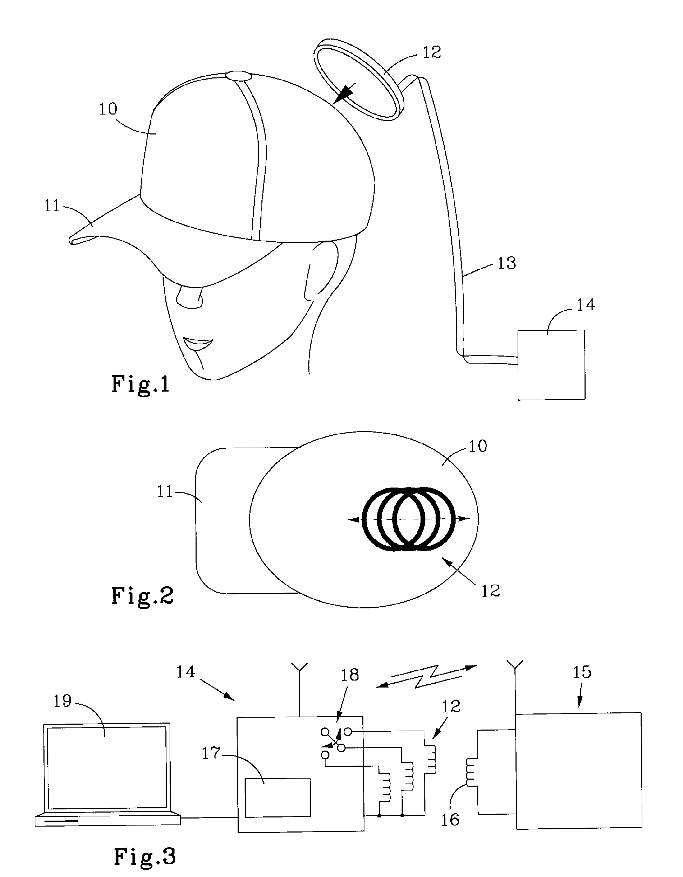

Comme le montre la figure 1, le dispositif selon l'invention prend place sur la tête d'un patient équipé d'un implant crânien (non représenté) possédant une source d'énergie électrique, formée d'une batterie ou d'une super-capacité, qu'il y a lieu de recharger par transfert inductif transcutané d'énergie à une bobine secondaire associé à l'implant. Cette bobine est avantageusement circulaire et réalisée en cuivre ou tout autre matériau hautement conducteur.

Le dispositif est constitué d'une calotte 10 qui emboîte le crâne du patient. Le dessin montre que cette calotte est associée à une visière 11 pour former une casquette de type base-ball, très discrète et évitant d'attirer l'attention sur le patient. Bien entendu, la visière 11 peut être supprimée, la calotte 10 formant alors un simple bonnet.

La calotte 10 comporte, cousue en regard de l'implant crânien, une bobine primaire 12, par exemple en forme de cercle. Celle-ci n'est pas visible de l'extérieur, afin d'assurer au patient toute la discrétion qu'il peut souhaiter. Le dessin la montre donc extraite de la calotte.

Comme la bobine de l'implant, la bobine primaire 12 est réalisée en cuivre ou tout autre matériau hautement conducteur. Les essais effectués ont montré qu'un couplage satisfaisant est obtenu avec une bobine primaire 12 ayant un diamètre d'environ 40 mm et une bobine secondaire ayant un diamètre d'environ 20 mm, la distance qui les sépare étant de 10 à 30 mm.

La bobine primaire 12 est électriquement reliée par des fils 13, ressortant dans le bas de la nuque, à une station de base 14, éventuellement portée par le patient, qui commande son alimentation. Cette station comporte avantageusement, comme mentionné dans le document US 2005/0004619 déjà cité, une interface sur laquelle le patient peut agir pour activer, surveiller et terminer la recharge. La station de base peut aussi contenir d'autres composants permettant au patient, notamment par liaison RF, de connaître le niveau du courant de charge et l'indication que la source d'énergie de l'implant est complètement chargée.

Bien entendu, il peut être difficile, pour le patient, de toujours replacer sa casquette de manière à ce que les deux bobines sont bien centrées l'une sur l'autre afin d'assurer un couplage optimal. Ainsi, afin de raccourcir le plus possible le temps de recharge, la calotte 10 peut donc, comme le montre la figure 2, posséder une pluralité de bobines primaires 12 réparties sur une surface suffisamment grande pour que l'une de ces bobines se trouve en regard de la bobine de l'implant. La figure 2 montre que les bobines primaires sont décalées selon un seul axe, mais il va de soi que toute autre répartition peut se faire.

La figure 3 donne un schéma de principe des moyens utilisés pour identifier la bobine primaire la mieux centrée sur la bobine de l'implant. Sur cette figure, on a représenté en 15 l'implant crânien et en 16 sa bobine.

La calotte 10 possède, par exemple, trois bobines primaires 12 qu'un microprocesseur 17 va, par l'intermédiaire d'un commutateur électronique 18, successivement solliciter afin d'identifier, grâce à la liaison RF entre l'implant 15 et la station de base 14, celle qui présente le meilleur couplage avec la bobine secondaire 16. La recharge est alors automatiquement lancée dans les conditions optimales.

Si le patient possède un ordinateur 19, il peut suivre sur son écran l'opération de sélection. Sinon, une simple diode (non représentée) disposée sur la station de base 14 peut l'informer qu'une recharge optimisée est en cours.

La présente description a été faite en se référant à des bobines circulaires, mais il va de soi qu'elles peuvent avoir toute autre forme, choisie par l'homme de métier, pour accomplir au mieux la fonction désirée.

Ainsi est proposé un dispositif de recharge de la source d'énergie d'un implant crânien. Ce dispositif est peu coûteux, simple à utiliser, confortable, discret et assure une recharge aussi courte que possible.

## Revendications

1. Dispositif de recharge de la source d'énergie électrique d'un implant crânien par transfert inductif transcutané d'énergie entre au moins une bobine primaire (12) et une bobine secondaire associée à l'implant, ledit dispositif comportant , en plus de la bobine primaire, des moyens pour la positionner et la maintenir en regard de la bobine secondaire et une station de base (14) pour commander son alimentation, **caractérisé en ce que** lesdits moyens sont constitués par une calotte (10) destinée à emboîter le crâne du patient.

2. Dispositif selon la revendication 1, **caractérisé en ce que** la bobine primaire (12) est en forme de cercle.

3. Dispositif selon l'une des revendications 1 et 2, **caractérisé en ce que** ladite calotte (10) est associée à une visière (11) pour former une casquette de type base-ball.

4. Dispositif selon l'une des revendications 1 à 3, **caractérisé en ce que** la bobine primaire (12) est cousue dans la calotte en regard de l'implant.

5. Dispositif selon l'une des revendications 1 à 4, **caractérisé en ce q**'il comporte une pluralité de bobines primaires (12) réparties sur une surface suffisamment grande pour que l'une de ces bobines se trouve, de manière optimale, en regard de la bobine de l'implant.

6. Dispositif selon la revendication 5, **caractérisé en ce que** ladite station de base (14) comporte des moyens permettant, grâce à une communication sans fil avec l'implant, de sélectionner et activer la bobine primaire (12) la mieux couplée avec sa bobine.
